# EUROPÄISCHE PATENTSCHRIFT

(11) **EP 3 335 687 B1**
(45) Veröffentlichungstag und Bekanntmachung des Hinweises auf die Patenterteilung: **12.02.2020**
(21) Anmeldenummer: 16204527.2
(22) Anmeldetag: 15.12.2016
(51) Int. Cl.: A61K 6/00, A61K 6/083

(54) **AUSBRENNBARES DENTALES MODELLIERMATERIAL**
FIREABLE DENTAL MODELLING MATERIAL
MATIÈRE À MODELER DENTAIRE CALCINABLE

(43) Veröffentlichungstag der Anmeldung: 20.06.2018
(73) Patentinhaber: Ivoclar Vivadent AG, 9494 Schaan (LI)
(72) Erfinder: BONDERER, Lorenz Josef, 7320 Sargans (CH)
(74) Vertreter: Uexküll & Stolberg

(56) Entgegenhaltungen:
- DE-A1- 19 900 459
- US-A- 4 613 560
- US-A1- 2010 029 801
- US-A1- 2014 183 799

## Beschreibung

Die vorliegende Erfindung betrifft Materialien, die sich besonders zur Herstellung von dentalen Modellen für Ausschmelzverfahren eignen. Ausschmelzverfahren werden zur Herstellung von Formteilen eingesetzt, die eine hohe Genauigkeit erfordern, und damit insbesondere zur Herstellung von dentalen Restaurationen, wie Inlays, Onlays, Veneers, Kronen, Brücken und Gerüsten.

Zur Herstellung von Dentalrestaurationen nach dem klassischen Wachsausschmelzverfahren (Lost-Wax Technik) wird ein Wachsmodell (Wax-up) geformt, in eine Einbettmasse eingebettet und nach deren Abbinden in einem Ofen aufgeheizt. Das Wachs schmilzt auf und fließt aus der Gießform. Anschließend wird die Gießform weiter erhitzt, wobei Reste an Wachs verbrennen. Auf diese Weise wird ein Negativ des Wachsmodells erhalten, in das anschließend eine Legierung oder ein Keramikmaterial vergossen wird. Das Gussobjekt ist dann wieder das Positiv, das der Wachsmodellation exakt gleicht.

Die Herstellung des Wachsmodells kann auf unterschiedliche Weise erfolgen. Bei der manuellen Modellierung wird vom Zahntechniker ein Modell der Zahnrestauration mit einem speziellen Dentalwachs schichtweise auf einem Gipsmodell aufgebaut. Die Wachse erweichen beim Erwärmen und fließen nach dem Schmelzen in der Regel ohne Risse zu verursachen aus der Einbettmasse heraus. Dieses Verfahren ist nach wie vor der Goldstandard hinsichtlich der Qualität von Guss- und Pressobjekten.

Bei neueren Verfahren wird das Wachsmodell mittels CAD-CAM-Verfahren aus Wachsscheiben oder -blöcken herausgefräst. Die hierbei verwendeten Wachse erfordern einen Kompromiss zwischen Fräsbarkeit und Ausbrennbarkeit. Die heute kommerziell erhältlichen Produkte erfüllen meist beide Anforderungen hinreichend gut. Auch diese Wachse erweichen beim Erwärmen und fließen nach dem Schmelzen ohne Risse zu verursachen aus der Einbettmasse heraus.

Wachsmodelle sind relativ empfindlich, so dass zunehmend alternative Werkstoffe zur Herstellung von Modellen eingesetzt werden. Bei CAD-CAM Verfahren werden beispielsweise Kunststoffscheiben und -blöcke sowie Kunststoff-Wachs-Hybridscheiben verwendet. Bei der manuellen Modellierung werden als Alternative zu Wachsen härtbare Harze eingesetzt, die sich kalt durch das Mischen von zwei Komponenten, thermisch oder bevorzugt durch Licht härten lassen. Diese Materialien enthalten relativ große Mengen an nicht schmelzbaren Duromeren.

In jüngerer Zeit werden zur Herstellung von Modellen vermehrt sogenannte generative Verfahren eingesetzt. Hierunter werden Fertigungsverfahren verstanden, bei denen aus computergestützten Konstruktionsdaten (CAD-Daten) dreidimensionale Modelle oder Bauteile hergestellt werden (A. Gebhardt, Vision of Rapid Prototyping, Ber. DGK 83 (2006) 7-12). Dabei handelt es sich um Verfahren, wie z.B. die Stereolithographie (SL), selektives Lasersintern (SLS), 3-D-Printing, Fused Deposition Modelling (FDM), Ink-Jet-Printing (IJP), 3D-Plotting, Multi-Jet Modelling (MJM), Solid Freeform Fabrication (SFF), Laminated Object Manufacturing (LOM), Laser Powder Forming (LPF), mit denen sich Modelle, Bau- oder Formteile auch in Kleinserie kostengünstig herstellen lassen (A. Gebhardt, Generative Fertigungsverfahren, 3. Aufl., Carl Hanser Verlag, München 2007, 77ff.). Bei der Stereolithographie wird ein Formteil auf der Basis von CAD-Daten schichtweise aus einem flüssigen und härtbaren Monomerharz aufgebaut (A. Beil, Fertigung von Mikro-Bauteilen mittels Stereolithographie, Düsseldorf 2002, VDI-Verlag 3 ff.). Generative Verfahren werden häufig unter dem Begriff "Rapid Prototyping" (RP) zusammengefasst.

Nachteilig an den als Alternative zu Wachsen verwendeten Materialien ist ihr Schmelzverhalten. Reine Wachse erweichen beim Erwärmen zunächst, schmelzen dann und fließen anschließend aus der Form heraus. Sie üben daher bei der thermisch bedingten Ausdehnung keine großen Kräfte auf das Einbettmaterial aus. Die Alternativwerkstoffe zeigen dieses Schmelzverhalten nicht. Sie dehnen sich beim Abbinden/Aushärten der Einbettmasse und dem damit verbundenen Temperaturanstieg sowie beim anschließenden Erhitzen thermisch aus, fließen dabei aber nicht aus der Form heraus sondern werden hauptsächlich durch thermische Zersetzung daraus entfernt. Beim Ausdehnen üben sie daher Druck auf das Einbettmaterial aus, was zu Spannungen und insbesondere bei großvolumigen Dentalrestaurationen zu Rissen in der Negativform führt, die Press- und Gussfahnen und bei größeren Rissen auch Fehlpressungen oder Fehlgüsse zur Folge haben.

Es ist bekannt, härtbaren Materialien Wachspartikel zuzumischen. Diese sollen beim Abbinden der Einbettmasse oder beim Ausbrennen im Ofen schmelzen und so Raum für das sich ausdehnende Material schaffen. Allerdings kann das geschmolzene Wachs häufig nicht oder nur schlecht entweichen, weil es von dem polymerisierten Material eingeschlossen wird und weil der Diffusionskoeffizient der geschmolzenen Wachsmoleküle durch die Polymermatrix hindurch nur sehr klein ist. Im ungünstigsten Fall wird durch die große Volumenzunahme der Wachse beim Schmelzen die thermische Ausdehnung des Modells sogar erhöht, statt sie zu reduzieren.

Die US 2010/0029801 A1 offenbart Schlicker zur Herstellung keramischer Formteile, die 5 bis 65 Gew.-% polyreaktives Bindemittel, 0,001 bis 1,0 Gew.-% Photoinitiator und 35 bis 90 Gew.-% Keramik- oder Glaskeramikpartikel enthalten. Die Schlicker können als optionale Komponente einen Weichmacher in einer Menge von 0 bis 15 Gew.-% bezogen auf das polyreaktive Bindemittel enthalten, wie z.B. Polyethylenglykol.

Die US 2014/0183799 A1 offenbart Schlicker zur Herstellung von Keramikbauteilen, die zusätzlich zu den in US 2010/0029801 A1 genannten Komponenten mindestens ein saures, radikalisch polymerisierbares Monomer enthalten. Die Schlicker zeichnen sich durch eine gute Haftung zwischen den stereolithographisch erzeugten Schichten aus.

Der Erfindung liegt die Aufgabe zugrunde, Materialien zum Modellieren von Dentalprothesen zur Verfügung zu stellen, die die oben genannten Nachteile nicht aufweisen. Sie sollen die Herstellung von Modellen erlauben, insbesondere durch stereolithographische Verfahren, die sich nach dem Abbinden der Einbettmasse ohne Rissbildung entfernen lassen, so dass fehlerfreie Gießformen und Restaurationen erhalten werden.

Erfindungsgemäß wird diese Aufgabe durch radikalisch polymerisierbare Zusammensetzungen gelöst, die (a) mindestens ein radikalisch polymerisierbares Monomer, (b) mindestens einen Initiator für die radikalische Polymerisation und (c) mindestens eine inerte Komponente enthalten. Bei der Komponente (c) handelt es sich um eine organische Verbindung.

Die erfindungsgemäßen Zusammensetzungen sind dadurch gekennzeichnet, dass sie 20 bis 90 Gew.-% radikalisch polymerisierbares Monomer (a), 0,1 bis 5 Gew.-% Initiator (b) und 9,9 bis 79,9 Gew.-% inerte Komponente (c) enthalten, jeweils bezogen auf die Gesamtmasse der Zusammensetzung, und dass sie als Komponente (a) mindestens ein (Meth)acrylat und/oder (Meth)acrylamid und als inerte Komponente (c) ein oder mehrere Polyethylenglykole (PEG) mit einem Molekulargewicht von 1000 bis 5000 g/mol, bevorzugt 1000 bis 3000 g/mol, Polypropylenglykole (PPG) mit einem Molekulargewicht von 1000 bis 4000 g/mol, bevorzugt 1500 bis 3000 g/mol und insbesondere 2000 g/mol, PEG-PPG-Co-Polymere mit einem Molekulargewicht von 1000 bis 10000 g/mol, vorzugsweise 1500 bis 5000 g/mol und insbesondere 2000 bis 4000 g/mol, Ethylendiamintetrakispropoxylate und/oder Ethylendiamintetrakisethoxylate mit einem Molekulargewicht von 1000 bis 10000 g/mol, insbesondere von 1000 bis 5000 g/mol enthalten.

In allen Fällen sind die propoxylierten Verbindungen gegenüber den ethoxylierten Verbindungen bevorzugt, weil Ethoxylate bei höheren Molekulargewichten dazu tendieren, fest zu werden und dann höhere Verarbeitungstemperaturen benötigen.

Die Komponente (c) kann bei Raumtemperatur fest sein, solange sie bei der Verarbeitungstemperatur nicht aus dem verwendeten Monomer bzw. aus der Monomermischung ausfällt. Bevorzugt sind jedoch Stoffe, die bei Raumtemperatur pastös und insbesondere flüssig sind. Die Konsistenz der Komponente (c) lässt sich beispielsweise durch den Anteil an PPG-Gruppen einstellen, z.B. durch das Mischen von propoxylierten und ethoxylierten Verbindungen oder durch das Erhöhen des Anteils an PPG-Gruppen innerhalb des Moleküls, z.B. bei PEG-PPG-Copolymeren. Der Anteil an PPG-Gruppen ist vorzugsweise so hoch, dass die Komponente flüssig oder pastös ist, besonders bevorzugt so hoch, dass der Pourpoint kleiner als die Verarbeitungstemperatur ist.

Der Pourpoint bezeichnet nach DIN 51597 für ein flüssiges Produkt die Temperatur, bei der es bei Abkühlung gerade noch fließt. Der Stockpunkt nach DIN 51583 bezeichnet die Temperatur, bei der das zuvor flüssige Produkt erstarrt. Die Bestimmung des Pourpoints erfolgt nach DIN 51597/ISO 3016. Ethylendiamintetrakispropoxylate und Mischungen aus Ethylendiamintetrakisethoxylaten und -propoxylaten sind gegenüber den Ethylendiamintetrakisethoxylaten bevorzugt.

Wenn nicht anders angegeben, handelt es sich hier bei der Molmasse von Polymeren in allen Fällen um die durch Viskosimetrie bestimmte Molmasse Mη (Viskositätsmittel).

Besonders bevorzugte Komponenten (c) sind Polypropylenglykole (PPG), insbesondere PPG mit einem Molekulargewicht von 1000 bis 4000 g/mol, vorzugsweise 1500 bis 3000 g/mol, Co-PEG-PPG mit einem Molekulargewicht von 1000 bis 10000 g/mol, vorzugsweise 1500 bis 5000 g/mol und ganz besonders Polypropylenglycol mit einem Molekulargewicht von ca. 2000 g/mol.

Es können vorteilhaft auch Mischungen der genannten Stoffe eingesetzt werden. Neben den genannten Komponenten können diese Mischung in einer untergeordneten Menge auch Verbindungen mit einem Molekulargewicht von weniger als 1000 g/mol enthalten.

Endständige -OH Gruppen der genannten Verbindungen können verestert oder verethert sein, beispielsweise mit Methyl-, Ethyl-, Propyl- oder iso-Propylgruppen bzw. Formiat-, Acetat-, Propionat- oder iso-Propionatgruppen.

Die Komponente (c) ist unter Normalbedingungen (20°C, 1013 mbar) flüssig oder fest und hat vorzugsweise einen Pourpoint von mehr als -150°C, besonders bevorzugt von mehr als -100°C und ganz besonders bevorzugt von mehr als -50°C.

Die Komponente (c) hat vorzugsweise einen Siedepunkt von über 80°C, bevorzugt über 120°C, besonders bevorzugt über 150°C. Komponenten, die unter Normaldruck nicht ohne Zersetzung sieden, haben vorzugsweise einen thermische Zersetzungstemperatur von über 120°C, bevorzugt über 135°C, besonders bevorzugt über 150°C. Durch die relativ hohe Siede- bzw. Zersetzungstemperatur wird sichergestellt, dass die Komponente (c) auch unter Anwendungsbedingungen fest bzw. flüssig ist und beispielsweise beim verdrucken oder beim Abbinden der Einbettmasse nicht verdampft. Verbindungen mit einer Siedetemperatur von maximal 350°C und/oder einer Zersetzungstemperatur von maximal 500°C sind bevorzugt.

Die Komponente (c) ist chemisch inert, d.h., dass sie unter Lager- und Anwendungsbedingungen nicht oder nicht merklich mit den übrigen Komponenten des Materials reagiert. Insbesondere enthält die Komponente (c) keine radikalisch polymerisierbaren Gruppen und copolymerisiert daher nicht mit dem Monomer (a). Sie ist mit der Komponente (a) homogen mischbar, und Mischungen von (a) und (c) sind bei der Verarbeitungstemperatur stabil, d.h. es findet keine Phasentrennung statt, insbesondere fällt oder kristallisiert die Komponente (c) bei der Verarbeitung nicht aus. Die Verarbeitung der erfindungsgemäßen Werkstoffe, d.h. die Herstellung von Modellen, beispielsweise durch stereolithographisches Verdrucken, erfolgt vorzugsweise bei einer Temperatur von 15 bis 35°C, vorzugsweise 15 bis 30°C. Bevorzugt sind Werkstoffe, die bei Raumtemperatur (20°C) verarbeitet und gelagert werden können, so dass die Materialien zur Verarbeitung nicht zusätzlich erwärmt werden müssen oder zur Lagerung nicht gekühlt werden müssen.

Als Komponente (c) kommen besonders rückstandsfrei verbrennbare Verbindungen in Betracht. Hierunter werden Stoffe verstanden, die bei der Verbrennung vorzugweise weniger als 0,1 Gew.-% Asche bilden. Bevorzugt sind Verbindungen, die beim Erhitzen in einer oxidierenden Atmosphäre auf eine Temperatur von ca. 850°C verbrennen.

Die erfindungsgemäßen Zusammensetzungen enthalten als Komponente (a) mindestens ein radikalisch polymerisierbares Monomer und als Komponente (b) mindestens einen Initiator für die radikalische Polymerisation. Sie liegen vorzugswiese in Form einer homogenen Mischung der Komponenten (a), (b), (c) und ggf. vorhandener optionaler Bestandteile vor. Sie können in Abhängigkeit vom verwendeten Initiator thermisch, über ein Zweikomponenten-Initiatorsystem oder photochemisch gehärtet werden. Zusammensetzungen, die einen Photoinitiator enthalten, sind bevorzugt.

Erfindungsgemäß sind Materialien zur Herstellung von Modellen durch manuelle Modellierung, besonders durch generative Fertigungsverfahren und ganz besonders durch Stereolithographie bevorzugt. Unter einem Modell wird hier insbesondere die gewünschte Positiv-Form für Ausschmelzverfahren verstanden. Anders als bei dem klassischen Wachsausschmelzverfahren wird das Modell nach dem Abbinden der Einbettmasse zwar primär durch Ausbrennen aus der Form entfernt, dennoch wird das Verfahren hierin als Ausschmelzverfahren bezeichnet. Das erfindungsgemäße Material eignet sich besonders zur Herstellung von Modellen zur Anfertigung von Dentalrestaurationen.

Die erfindungsgemäßen Modelliermaterialien zeichnen sich durch eine relativ geringe maximale lineare thermische Ausdehnung aus. Vorzugsweise liegt diese unter 1,5%, bevorzugt unter 1%, besonders bevorzugt unter 0,7%. Die lineare thermische Ausdehnung wird im Temperaturbereich von 30°C bis 800°C gemessen, wobei die meisten Materialen bereits vor dem Erreichen von 800°C beginnen, sich zu zersetzten. Die Zersetzung äußert sich in einer Schrumpfung der Messkörper.

Besonders vorteilhat ist, dass die maximalen Ausdehnung bei einer Temperatur von unter 150°C, vorzugsweise unter 120°C, besonders bevorzugt unter 100°C und ganz besonders bevorzugt unter 90°C erreicht wird. Kommerziell erhältliche ausbrennbare SL-Harze weisen demgegenüber typischerweise eine maximale thermische Ausdehnung von 2% oder mehr auf, die zudem erst bei relativ hohen Temperaturen erreicht wird.

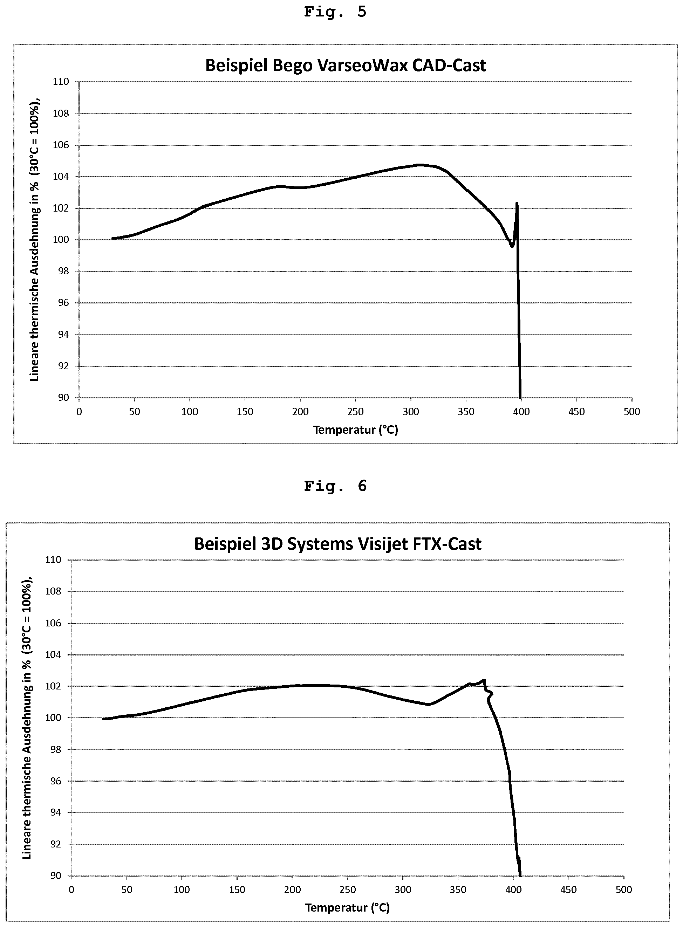

Figur 5 zeigt das thermische Ausdehnungsverhalten eines herkömmlichen Werkstoffs. Herkömmliche Materialien dehnen sich beim Erwärmen thermisch aus, und das Volumen nimmt kontinuierlich zu. Oberhalb einer bestimmten Temperatur beginnen sich die Materialien zu zersetzen. Die Zersetzung äußert sich in einer Volumenabnahme. Die Zersetzungstemperatur ist materialabhängig und liegt für die meisten organischen Werkstoffe bei über 300°C.

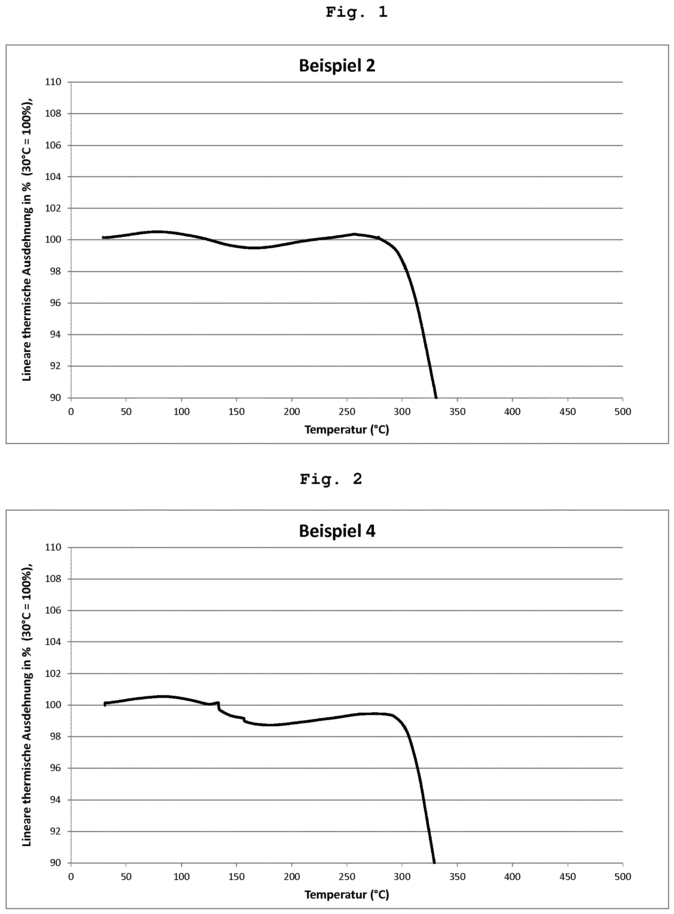

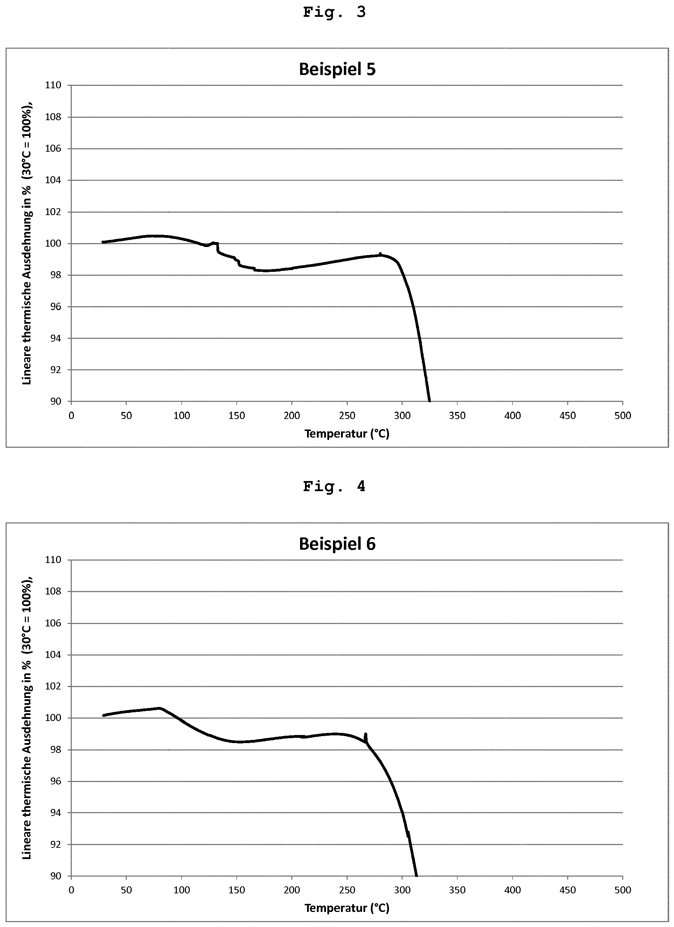

Erfindungsgemäße Materialien zeigen beim Erwärmen zunächst meist ebenfalls eine geringe Ausdehnung, die aber deutlich geringer als bei herkömmlichen Materialien ausfällt (Fig. 1-4). Die Ausdehnungskurven fallen bereits vor dem Erreichen der Zersetzungstemperatur wieder deutlich ab. Teilweise wird zwar bei höheren Temperaturen nochmals ein leichter Anstieg der Kurven beobachtet, dieser fällt aber vergleichsweise gering aus. Insgesamt dehnen sich die erfindungsgemäßen Werkstoffe über den gesamten Temperaturbereich bis zur Zersetzung deutlich weniger aus als bekannte Materialien.

Die Ausdehnungskurven durchlaufen ein Maximum. Dieses Maximum der thermischen Ausdehnung wird hier als maximale lineare thermische Ausdehnung bezeichnet.

Die Bestimmung der thermischen Ausdehnung erfolgt vorzugsweise an Zylindern mit 6 mm Durchmesser und 6 mm Höhe im Temperaturbereich von 30°C bis 800°C bei einer Heizrate von 5 K/min in einer Luftatmosphäre. Die Messsonde wird mit einer Anpresskraft von 0,1 N auf die Probe aufgelegt. Es wird die lineare Ausdehnung der Zylinder während des Aufheizens und der thermischen Zersetzung gemessen. Die Messung kann z.B. mit einem thermomechanischen Analysator (TMA) des Typs Q400 der Firma TA Instruments mit Macro-Expansion Tool erfolgen. Die Länge des Zylinders bei 30°C wird als Ursprungslänge, d.h. 0% Ausdehnung, angenommen.

Durch das thermische Ausdehnungsverhalten der erfindungsgemäßen Materialien wird das Risiko von Defekten wie Rissen oder gar der Zerstörung der Form während des Ausbrennens zumindest stark reduziert und meist ganz eliminiert. Die erfindungsgemäßen Materialien ergeben auf diese Weise rissfreie Gießformen mit hoher Genauigkeit und Präzision.

Die erfindungsgemäßen Materialien enthalten als radikalisch polymerisierbares Monomer (a) mindestens ein (Meth)acrylat und/oder (Meth)acrylamid, vorzugsweise ein oder mehrere mono- oder multifunktionelle (Meth)acrylate oder eine Mischung davon. Besonders bevorzugt sind Werkstoffe, die als radikalisch polymerisierbares Monomer mindestens ein multifunktionelles (Meth)acrylat oder eine Mischungen von mono- und multifunktionellen (Meth)acrylaten enthalten. Unter monofunktionellen (Meth)acrylaten werden Verbindungen mit einer, unter polyfunktionellen (Meth)acrylaten Verbindungen mit zwei oder mehr, vorzugsweise 2 bis 6 radikalisch polymerisierbaren Gruppen verstanden. Methacrylate sind in allen Fällen gegenüber den Acrylaten bevorzugt.

Besonders geeignete mono- oder multifunktionelle (Meth)-acrylate sind Methyl-, Ethyl-, 2-Hydroxyethyl-, Butyl-, Benzyl-, Tetrahydrofurfuryl- oder Isobornyl(meth)acrylat, p-Cumyl-phenoxyethylenglycolmethacrylat (CMP-1E), Bisphenol-A-di(meth)acrylat, Bis-G(M)A (ein Additionsprodukt aus (Meth)-acrylsäure und Bisphenol-A-diglycidylether), ethoxy- oder propoxyliertes Bisphenol-A-di(meth)acrylat, wie z.B. Bisphenol-A-di(meth)acrylat mit 3 (SR-348c = Methacrylat; SR-349 = Acrylat, Fa. Sartomer) oder 2 Ethoxygruppen (SR-348L = Methacrylat, Fa. Sartomer), 2,2-Bis[4-(2-(meth)acryloxypropoxy)phenyl]propan, UD(M)A (ein Additionsprodukt aus 2-Hydroxyethyl(meth)acrylat und 2,2,4- oder 2,4,4-Trimethylhexamethylen-1,6-diisocyanat), Di-, Tri-, Tetra-, Penta-, Hexa- oder Hepta-ethylenglycoldi(meth)acrylat, Di-, Tri-, Tetra-, Penta-, Hexa- oder Hepta-propylenglycoldi(meth)acrylat, Trimethylolpropantri(meth)acrylat, ethoxyliertes oder propopoxyliertes Trimethylolpropantri(meth)acrylat, z.B. 3-fach propoxyliertes Trimethylolpropantriacrylat (Sartomer SR-492,) und Tripropylenglycoldiacrylat, Pentaerythrittetra(meth)-acrylat, sowie Glycerindi- und -tri(meth)acrylat, 1,4-Butandioldi(meth)acrylat, 1,10-Decandioldi(meth)acrylat (D₃MA), 1,12-Dodecandioldi(meth)acrylat oder oligomere Polyether-, Polyester-, Epoxy-, Urethan-(meth)acrylate und Tricyclodecandimethanoldi(meth)acrylat.

Besonders bevorzugt sind mono- und insbesondere di- und trifunktionelle Acrylate und Methacrylate mit einem Molekulargewicht von <1000 g/mol, wie z.B. 2-Phenoxyethyl(meth)acrylat, aliphatische Urethan-diacrylate, Phthalsäure-HEA-Ester (Photomer 4173), Pyromellitsäure-di-HEA-Ester (HEA = 2-Hydroxyethylacrylat), Bis-G(M)A (ein Additionsprodukt aus (Meth)acrylsäure und Bisphenol-A-diglycidylether), 2,2-Bis[4-(2-(meth)acryloxypropoxy)phenyl]propan, UD(M)A, Triethylenglycoldi(meth)acrylat (TEGD(M)A) und 2-Phenoxethyl(meth)-acrylat (Acrylat SR339C Fa Sartomer/ Arkema). Diese Monomere zeichnen sich durch eine hohe Reaktivität, einen hohen Doppelbindungsumsatz, gute mechanische Eigenschaften, einen geringen Polymerisationsschrumpf und eine relativ niedrige Viskosität aus. Ganz besonders bevorzugt sind solche Materialen, die als Komponente (a) UDMA, TEGDMA, Bis-GMA (Additionsprodukt aus Methacrylsäure und Bisphenol-A-diglycidylether) oder 2-Phenoxyethylacrylat (SR339C, Fa. Sartomer/Arkema), eine Mischung von UDMA, TEGDMA und 2-Phenoxythylacrylat oder vorzugsweise UDMA, TEGDMA und Bis-GMA und insbesondere eine Mischung von UDMA und TEGDMA enthalten.

Die Eigenschaften der Werkstoffe vor und nach der Härtung lassen sich durch eine gezielte Kombinationen der Monomeren beeinflussen. Mischungen aus monofunktionellen und difunktionellen Monomeren zeichnen sich durch eine relativ geringe Viskosität und Reaktivität der Harzmischung aus, wobei Viskosität und Reaktivität mit dem Gehalt an monofunktionellen Monomeren abnehmen. Ein Gehalt an monofunktionellen Monomeren gewährleistet eine geringere Steifigkeit der durch Härtung der Materialien erhaltenen Modelle. Mischungen aus difunktionellen und trifunktionellen Monomeren weisen eine höhere Reaktivität auf, wobei die Reaktivität mit dem Gehalt an trifunktionellen Monomeren zunimmt. Der Gehalt an trifunktionellen Monomeren bewirkt eine höhere Sprödigkeit. Reaktivität und Viskosität der Harzmischung sowie der Polymerisationsschrumpf werden außerdem durch die Molmasse der Monomeren bestimmt, wobei mit zunehmender Molmasse der Polymerisationsschrumpf abnimmt, während die Viskosität ansteigt.

Bevorzugte Photoinitiatoren (b) zur Initiierung der radikalischen Photopolymerisation sind Benzophenon, Benzoin sowie deren Derivate oder α-Diketone oder deren Derivate, wie 9,10-Phenanthrenchinon, 1-Phenyl-propan-1,2-dion, Diacetyl oder 4,4'-Dichlorbenzil. Besonders bevorzugt werden Campherchinon (CQ) und 2,2-Dimethoxy-2-phenyl-acetophenon und ganz besonders bevorzugt α-Diketone in Kombination mit Aminen als Reduktionsmittel eingesetzt, wie z.B. 4-(Dimethylamino)-benzoesäureester (EDMAB), N,N-Dimethylaminoethylmethacrylat, N,N-Dimethyl-sym.-xylidin oder Triethanolamin. Weiter bevorzugt sind Diethylthioxanthen (DETX, CAS 82799-44-8) und Isopropylthioxanthon (ITX, CAS 75081-21-9), beide jeweils vorzugsweise in Kombination mit Ethyl-4-(dimethylamino)benzoat (EMBO, CAS Nr. 10287-53-3). Ebenso bevorzugt sind [1-(4-Phenylsulfanylbenzoyl)-heptylidenamino]benzoat (Irgacure OXE 01), und [1[9-Ethyl-6-(2-methylbenzoyl)carbazol-3-yl]ethylidenamino]acetat (Irgacure OXE 02).

Besonders bevorzugte Photoinitiatoren sind weiterhin Norrish-Typ-I-Photoinitiatoren, vor allem Monoacyl- oder Bisacylphosphinoxide, und insbesondere Monoacyltrialkyl- bzw. Diacyldialkylgermanium-Verbindungen, wie z.B. Benzoyltrimethylgermanium, Dibenzoyldiethylgermanium oder Bis(4-methoxybenzoyl)diethylgermanium (MBDEGe). Dabei lassen sich vorteilhaft auch Mischungen der verschiedenen Photoinitiatoren einsetzen, wie z.B. Bis(4-methoxybenzoyl)diethylgermanium in Kombination mit Campherchinon und 4-Dimethylaminobenzoesäureethylester.

Ganz besonders bevorzugt sind Campherchinon (CAS Nr. 10373-78-1) in Kombination mit Ethyl-4-(dimethylamino)benzoat (EMBO, CAS Nr. 10287-53-3) sowie Phenylbis(2,4,6-trimethyl¬benzoyl)¬phos-phin¬oxid (Irgacure 819, CAS 162881-26-7), Diphenyl (2,4,6-tri¬methyl¬benzoyl)¬ phos¬phinoxid (TPO, CAS Nr. 75980-60-8), 2-Benzyl-2-(dimethylamino)-4'-morpho¬lino¬butyro¬phenon (Irgacure 369, CAS Nr. 119313-12-1), 1-Buta¬non-2-(dimethylamino)-2-(4-methylphenyl)methyl-1-4-(4-mor¬pho¬linyl)¬phenyl (Irgacure 379, CAS-Nr. 119344-86-4) und ganz besonders Bis(4-meth¬oxy-benzoyl)di¬ethyl¬ger¬manium (MBDEGe; Ivocerin).

Erfindungsgemäß wurde überraschend gefunden, dass durch eine Abstimmung der Komponenten das thermische Ausdehnungsverhalten der Materialien gezielt so eingestellt werden kann, dass ihre maximale thermische Ausdehnung unter 1,5%, bevorzugt unter 1%, besonders bevorzugt unter 0,7% liegt, und gemäß einer besonders bevorzugten Ausführungsform die maximale Ausdehnung bei einer Temperatur von unter 150°C, vorzugsweise unter 120 °C, besonders bevorzugt unter 100°C und ganz besonders bevorzugt unter 90°C erreicht wird.

Es wurde gefunden, dass die erfindungsgemäß verwendete, inerte Komponente (c) und insbesondere die genannten bevorzugten Komponenten mit radikalisch polymerisierbaren Monomeren und insbesondere mit Acrylaten und Methacrylaten homogen mischbar sind und die Polymerisation dieser Monomere nicht beeinträchtigen. Bei der stereolithographischen Verarbeitung der erfindungsgemäßen Zusammensetzungen im flüssigen Zustand können dadurch eine hohe Reaktivität sowie kurze Belichtungs- und Prozesszeiten gewährleistet werden.

Erfindungsgemäß sind solche Materialen bevorzugt, bei denen die Komponente (a) UDMA, TEGDMA oder eine Mischung davon enthält. Besonders bevorzugt sind Materialen, in denen die Komponente (a) neben UDMA und/oder TEGDMA maximal 20 Gew.-%, vorzugsweise maximal 10 Gew.-%, besonders bevorzugt maximal 5 Gew.-% und ganz besonders bevorzugt weniger als 3 Gew.-% oder keine weiteren Monomere enthält, wobei sich die Prozentangaben auf die Gesamtmasse der Komponente (a) beziehen. Bevorzugt sind insbesondere solche Materialen, die als Komponente (a) UDMA oder TEGDMA, besonders bevorzugt eine Mischung von UDMA, TEGDMA und SR339C oder von UDMA, TEGDMA und Bis-GMA und insbesondere eine Mischung von UDMA und TEGDMA enthalten. Als Komponente (c) ist jeweils PPG mit einem Molgewicht von 1000 bis 4000 g/mol, vorzugsweise von 1500 bis 3000 g/mol, ganz besonders bevorzugt 2000 g/mol bevorzugt.

Im Fall der erfindungsgemäß bevorzugten Monomere stellt PPG mit einem Molgewicht von 2000 g/mol einen guten Kompromiss dar. Im Fall von PEG-PPG-PEG ist ein Molekulargewicht von ca. 3500 g/mol vorteilhaft. Durch die Verwendung dieser Komponente wird die Temperatur der maximalen thermischen Ausdehnung zu höheren Werten hin verschoben.

Neben den oben genannten Komponenten können die erfindungsgemäßen Materialien vorteilhaft weitere Additive enthalten. Für stereolithographische Anwendung sind Werkstoffe bevorzugt, die mindestens ein Farbmittel und vorzugsweise auch mindestens einen Polymerisationsinhibitor enthalten.

Als Farbmittel sind organische Farbstoffe und Pigmente bevorzugt, insbesondere Azofarbstoffe, Carbonylfarbstoffe, Cyaninfarbstoffe, Azomethine und Methine, Phthalocyanine und Dioxazine. Besonders bevorzugt sind Farbstoffe, die in den Materialien löslich sind, insbesondere Azofarbstoffe. Als Farbmittel eignen sich außerdem anorganische und insbesondere organische Pigmente, die sich gut in den Materialien dispergieren lassen. Bevorzugt sind Azopigmente und Nichtazopimente. Als Farbstoffe werden vorzugsweise solche Stoffe eingesetzt, die rückstandsfrei ausbrennen. Aus diesem Grund sind organische gegenüber anorganischen Pigmenten bevorzugt. Falls anorganische Pigmente eingesetzt werden, wird ihre Menge vorzugsweise so bemessen, dass ihr Aschegehalt nach dem Ausbrennen unter 0,1 Gew.-% bezogen auf das Gesamtgewicht des ausbrennbaren Materials liegt.

Vorzugsweise enthalten die Materialien mindestens einen Farbmittel, das im gleichen Wellenlängenbereich wie der Polymerisationsinitiator absorbiert. Besonders bevorzugt sind Farbmittel, die ein Absorptionsmaximum aufweisen, das der Wellenlänge des zur Härtung eingesetzten Lichts entspricht. Sehr vorteilhaft sind Farbmittel mit einem Absorptionsmaximum im Bereich von 350 bis 550 nm, vorzugsweise 380 bis 480 nm.

Farbmittel verhindern, dass das zur Härtung eingesetzte Licht zu tief in die Materialien eindringt, und wirken sich so vorteilhaft auf die Präzision der Bauteile im Druckprozess aus, insbesondere bei stereolithographischen Verfahren. Darüber hinaus können Farbmittel auch zu ästhetischen Zwecken zugesetzt werden.

Der oder die Polymerisationsinhibitoren dienen als Stabilisator zur Verhinderung einer spontanen Polyreaktion. Die Inhibitoren bzw. Stabilisatoren verbessern die Lagerstabilität der Materialien und verhindern darüber hinaus eine unkontrollierte Polyreaktion in der stereolithographischen Wanne. Die Inhibitoren werden vorzugsweise in einer solchen Menge zugesetzt, dass die Materialen über einen Zeitraum von ca. 2-3 Jahre lagerstabil sind. Besonders bevorzugt werden die Inhibitoren in einer Menge von 0,001 bis 1,0 Gew.-%, ganz besonders bevorzugt 0,001 bis 0,20 Gew.-% eingesetzt, jeweils bezogen auf die Gesamtmasse des Materials.

Bevorzugt sind sogenannte aerobe Inhibitoren, z.B. Phenole, wie Hydrochinonmonomethylether (MEHQ) oder 2,6-Di-tert.-butyl-4-methyl-phenol (BHT), die nur in Gegenwart von Sauerstoff effektiv wirksam sind und vorzugsweise in einem Konzentrationsbereich von 100-2000 ppmw verwendet werden. Geeignete anaerobe Inhibitoren sind Phenothiazin, 2,2,6,6-Tetramethyl-piperindin-1-oxyl-Radikal (TEMPO), Iod und Kupfer-(I)-iodid. Diese wirken schon in geringen Konzentrationen von vorzugsweise 10-200 ppmw auch bei Abwesenheit von Sauerstoff. Eine Polymerisation findet erst dann statt, wenn diese Additive verbraucht sind. Dabei ist es von Vorteil, eine Mischung von aeroben und anaeroben Inhibitoren einzusetzen.

Aerobe Inhibitoren werden vorzugsweise in einer Menge von 0,001 bis 0,50 Gew.-% und anaerobe Inhibitoren in einer Menge von 0,001 bis 0,02 Gew.-% eingesetzt, jeweils bezogen auf die Gesamtmasse des Materials. Bevorzugte Mischungen enthalten 0,005-0,10 Gew.-% an aeroben Inhibitoren und 0,001- bis 0,02 Gew.-% an anaeroben Inhibitoren, ebenfalls bezogen auf die Gesamtmasse des Materials.

Die erfindungsgemäßen Modelliermaterielaien können außerdem partikulären Füllstoff enthalten. Generell sind als Füllstoffe organische Partikel bevorzugt, insbesondere Füllstoffe, die aschefrei verbrennen. Der oder die Füllstoffe haben vorzugsweise eine Partikelgröße von weniger als 25 µm, bevorzugt weniger als 10 µm und besonders bevorzugt weniger als 5 µm. Bei allen Partikelgrößen hierin handelt es sich, wenn nicht anders angegeben, um D50-Werte, d.h. 50 Vol.-% der Partikel haben einen Durchmesser, der kleiner als der angegebene Wert ist. Füllstoffe dienen primär dazu, die Viskosität, die mechanischen und/oder die optischen Eigenschaften der Werkstoffe einzustellen.

Die Oberfläche der Füllstoffe kann modifiziert werden, beispielsweise um die Dispergierbarkeit der Füllstoffe in der organischen Matrix zu verbessern. Zur Oberflächenmodifizierung werden vorzugsweise solche Verbindungen eingesetzt, die chemisch, d.h. durch ionische oder kovalente Bindungen an die Oberfläche der Füllstoffe gebunden werden. Bevorzugt sind Verbindungen, die entweder Säuregruppen, vorzugsweise Carbonsäure-, Phosphonsäure-, Hydrogenphosphatgruppen oder saure Phosphorsäureestergruppen, oder Silylgruppen, vorzugsweise Alkoxysilylgruppen enthalten. Die Partikeloberfläche kann teilweise oder vorzugsweise vollständig mit dem Modifizierungsmittel bedeckt sein. Bei den erfindungsgemäß verwendeten Modifizierungsmitteln handelt es sich um monomere Verbindungen. Als Oberflächenmodifizierungsmittel eigen sich besonders lineare Carbonsäuren, wie z.B. Ameisensäure, Essigsäure, Propionsäure, Octansäure, Isobuttersäure, Isovaleriansäure, Pivalinsäure, oder Phosphonsäuren, z.B. wie Methyl-, Ethyl-, Propyl-, Butyl-, Hexyl, Octyl- oder Phenylphosphonsäure. Als silylgruppenhaltige Verbindungen sind Silane wie Propyltrimethoxysilan, Phenyltrimethoxysilan, Hexyltrimethoxysilan, Octyltrimethoxysilan, Trimethylchlorsilan, Trimethylbromsilan, Trimethylmethoxysilan und Hexamethyldisilazan bevorzugt. Ganz besonders bevorzugte Oberflächenmodifizierungsmittel sind saure Phosphorsäureester, wie z.B. Dimethyl-, Diethyl-, Dipropyl-, Dibutyl-, Dipentyl-, Dihexyl-, Dioctyl- oder Di(2-ethylhexyl)phosphat. Die Oberflächenmodifizierungsmittel können auch radikalisch polymerisierbare Gruppen, beispielsweise (Meth)acrylatgruppen, aufweisen, die mit der Komponente (a) reagieren und so in das Polymernetzwerk eingebunden werden.

Bevorzugte Füllstoffe sind partikuläres Wachse, insbesondere Carnaubawachs, vorzugsweise mit eine Partikelgröße von 1 bis 10 µm, vernetzte Polymethylmethacrylat (PMMA)-Partikel, vorzugsweise mit einer Partikelgröße von 500 nm bis 10 µm, sowie Polyamid-12 Partikel, vorzugsweise mit einer Partikelgröße von 5 bis 10 µm. Für die Stereolithographie werden bevorzugt Füllstoffe eingesetzt, deren maximale Partikelgröße kleiner als die Dicke der stereolithographisch erzeugten Schichten ist. Bevorzugt sind Partikel mit einer maximalen Größe von 25 µm, vorzugsweise maximal 15 µm.

Erfindungsgemäß sind füllstofffreie Materialien bevorzugt.

Die rheologischen Eigenschaften der erfindungsgemäßen Materialien werden an den gewünschten Anwendungszweck angepasst. Materialen zur stereolithographischen Verarbeitung werden vorzugsweise so eingestellt, dass ihre Viskosität im Bereich von 50 mPa·s bis 100 Pa·s, bevorzugt 100 mPa·s bis 10 Pa·s, besonders bevorzugt 100 mPa·s bis 5 Pa·s liegt. Die Viskosität wird bei der gewünschten Verarbeitungstemperatur der Materialiens mit einem Kegel-Platte-Viskosimeter bestimmt (Scherrate 100/s). Die Verarbeitungstemperatur liegt vorzugsweise im Bereich von 10 bis 70°C, besonders bevorzugt 20 bis 30°C.

Werkstoffe zur manuellen Verarbeitung werden vorzugsweise so eingestellt, dass sie eine pastenförmige Konsistenz haben.

Zur Herstellung dünnflüssiger Materialen werden vorzugsweise flüssige Monomere und dünnflüssige Komponenten (c) eingesetzt, während zur Herstellung von Materialien mit hoher Viskosität z.B. auch feste Monomere und/oder dickflüssige oder feste Komponenten (c) verwendet werden können.

Die erfindungsgemäßen Materialien können außerdem vorzugsweise zusätzlich einen oder mehrere UV-Absorber enthalten. Bevorzugte UV-Absorber) sind 2,2'-Methylenbis[6-(2H-benzotriazol-2-yl)-4-(1,1,3,3-tetramethyl¬butyl)¬phenol] (CAS-Nr. 103597-45-1), 2,2',4,4'-Tetrahydroxybenzophenon (CAS-Nr. 131-55-5), 2-tert-Butyl-6-(5-chloro-2H-benzotriazol-2-yl)-4-methylphenol (CAS-Nr. 3896-11-5), 2,2'-Benzol-1,4-diyl¬bis(4h-3,1-benzoxazin-4-on) (CAS-Nr. 18600-59-4), 2-(4,6-Bis-(2,4-dimethylphenyl)-1,3,5-triazin-2-yl)-5-(octyl¬oxy)-phenol (CAS-Nr. 2725-22-6), 2-(2-Hydroxy-5-methylphenyl)benzotriazol (CAS-Nr. 2440-22-4) und 2-(2H-Benzotriazol-2-yl)-6-dodecyl-4-methyl¬phenol (CAS-Nr. 23328-53-2).

Ebenso geeignet sind sogenannte *Hindered Amine Light Stabilizers* wie Bis(1,2,2,6,6-pentamethyl-4-piperidyl)sebacat (CAS-Nr. 41556-26-7) und Methyl-1,2,2,6,6-pentamethyl-4-piperidylsebacat (CAS-Nr. 82919-37-7), Bis(1-octyloxy-2,2,6,6-tetramethyl-4-piperidyl)sebacat (CAS-Nr. 129757-67-1), Bis(1,2, 2,6,6-pentamethyl-4-piperidyl)-[[3,5-bis(1,1-dimethylethyl)-4-hydroxyphenyl]methyl]butylmalonat (CAS-Nr. 63843-89-0).

Neben den oben genannten Komponenten können die erfindungsgemäßen Materialien ein oder mehrere weitere Additive enthalten, die bevorzugt aus Verdickungsmitteln, optischen Aufhellern (z.B. Lumilux LZ Blue, CAS-Nr. 658084-50-5, oder 2,5-Bis(5-tert-butyl-benzoxazol-2-yl)thiophen, CAS-Nr. 7128-64-5) und im Fall füllstoffhaltiger Materialien aus Dispergiermitteln ausgewählt sind.

Die erfindungsgemäßen Materialien weisen vorzugsweise folgende Zusammensetzung auf:
- 42,5 bis 82,5 Gew.-%, besonders bevorzugt 50 bis 79,3 Gew.-% Komponente (a),
- 0,3 bis 2,5 Gew.-%, besonders bevorzugt 0,5 bis 1,5 Gew.-% (Photo-)Initiator (b) und
- 15 bis 55 Gew.-%, besonders bevorzugt 20 bis 48,5 Gew.-% inerte Komponente (c).

Eine ganz besonders bevorzugte Zusammensetzung enthält:
- 54,7 bis 77,7 Gew.-%, vorzugsweise 64 bis 74 Gew.-% Komponente (a), vorzugsweise UDMA, TEGDMA, eine Mischung von UDMA und TEGDMA oder eine Mischung von UDMA, TEGDMA und Bis-GMA,
- 0,5 bis 1,5 Gew.-%, vorzugsweise 0,6 bis 1,2 Gew.-% (Photo-)Initiator (b) und
- 23,8 bis 44,8 Gew.-%, vorzugsweise 24,8 bis 35,4 Gew.-% inerte Komponente (c), vorzugsweise PPG mit einem Molekulargewicht von 1000 bis 4000 g/mol, besonders bevorzugt 1500 bis 3000 g/mol, Co-PEG-PPG mit einem Molekulargewicht von 1000 bis 10000 g/mol, besonders bevorzugt 1500 bis 5000 g/mol, ganz besonders bevorzugt 2000 bis 4000 g/mol und insbesondere Polypropylenglycol mit einem Molekulargewicht von ca. 2000 g/mol.

Am meisten bevorzugt ist eine Zusammensetzung, die
- 64 bis 74 Gew.-% UDMA, TEGDMA, eine Mischung von UDMA und TEGDMA oder eine Mischung von UDMA, TEGDMA und Bis-GMA als Komponente (a),
- 0,6 bis 1,2 Gew.-% Photoinitiator (b) und
- 24,8 bis 35,4 Gew.-% Polypropylenglycol (PPG) mit einem Molekulargewicht von 1000 bis 4000 g/mol, besonders bevorzugt 1500 bis 3000 g/mol und insbesondere mit einem Molekulargewicht von ca. 2000 g/mol als inerte Komponente (c) enthält.

Weiterhin enthalten die erfindungsgemäßen Materialien vorzugsweise zusätzlich:
- 0,0001 bis 1 Gew.-%, vorzugsweise 0,0001 bis 0,5 Gew.-%, besonders bevorzugt 0,0001 bis 0,2 Gew.-% Farbmittel; und/oder
- 0,0001 bis 2 Gew.-%, vorzugsweise 0,0001 bis 1 Gew.-%, besonders bevorzugt 0,0001 bis 0,5 Gew.-% UV-Absorber; und/oder
- 0 bis 40 Gew.-%, vorzugsweise 0 bis 30 Gew.-%, besonders bevorzugt 1 bis 20 Gew.-% organischen Füller.

Außerdem enthalten die Materialien vorzugsweise
- 0 bis 5 Gew.-%, vorzugsweise 0 bis 3 Gew.-%, besonders bevorzugt 0 bis 2 Gew.-% weitere(s) Additiv(e).

Wenn nicht anders angegeben beziehen sich alle Angaben auf das Gesamtgewicht des Materials. Dabei werden die einzelnen Bestandteile vorzugsweise aus den oben genannten bevorzugten Komponenten ausgewählt.

Die erfindungsgemäßen Materialien eignen sich besonders zur Herstellung von Modellen für Gussarbeiten aus Metall oder Glaskeramik, insbesondere zur Herstellung von Modellen für Dentalrestauration, wie z.B. Inlays, Onlays, Veneers, Kronen, Brücken oder Gerüsten sowie abnehmbare (Teil-)Prothesen.

Gegenstand der Erfindung ist weiterhin ein Verfahren zur Herstellung von Dentalrestaurationen bei dem man
(A) mit einem erfindungsgemäßen Modelliermaterial ein Modell des zu restaurierenden Zahns oder der zu restaurierenden Zähne formt,
(B) man anschließend das Modell in eine Einbettmasse einbettet,
(C) man nach dem Abbinden der Einbettmasse das eigebettete Modell in einem Ofen soweit aufheizt, dass das Modelliermaterial vollständig aus der Gießform entfernt wird,
(D) man in das so hergestellte Gießform eine Legierung oder ein Glaskeramikmaterial gießt oder presst.

Die Herstellung des Modells in Schritt (A) erfolgt vorzugsweise durch ein stereolithographisches Verfahren. Hierzu wird durch die direkte oder indirekte Digitalisierung des zu restaurierenden Zahns bzw. der zu restaurierenden Zähne am Computer ein virtuelles Abbild der Zahnsituation erstellt, dann anhand dieses Abbilds am Computer ein Modell der Dentalrestauration konstruiert und dieses dann durch generative stereolithographische Fertigung hergestellt.

Anschließend kann das Modell in Schritt (B) auf herkömmliche Weise in eine Einbettmasse eingebettet werden. Die erfindungsgemäßen Materialien eignen sich zur Verwendung mit üblichen dentalen Einbettmassen. Man unterscheidet gipshaltige und gipsfreie Einbettmassen. Gipshaltige Einbettmassen enthalten als Hauptbestandteile Gips und Quarz im Verhältnis 1:2 bis 1:4. Gips dient als Bindemittel. Eine bevorzugte Quarzmodifikation ist Cristobalit. Bevorzugt sind gipsfreie Einbettmassen, die Phosphate als Bindemittel enthalten. Phosphatgebundene Einbettmassen enthalten hochtemperaturbeständige SiO₂-Modifikationen und einen Binder. Als Binder wird vorzugsweise ein Gemisch aus Magnesiumoxid und Ammoniumphosphat (NH₄H₂PO₄) verwandt. Die Massen binden nach dem Anmischen mit Wasser ab.

Das Entfernen des Modells aus der Gießform in Schritt (C) erfolgt durch Erhitzen z.B. in einem Ofen auf eine Temperatur von vorzugsweise 600°C bis 1000°C. Das Erhitzen kann ein- oder mehrstufig erfolgen. Es ist möglich aber nicht unbedingt erforderlich, die Temperatur kontrolliert auf die gewünschte Endtemperatur anzuheben. Anschließend wird die Gießform weiter erhitzt, wobei das restliche Modellmaterial thermisch zersetzt wird.

Nach dem Abbinden der Einbettmasse kann das eingebettete Modell direkt in einen Ofen gestellt werden, der auf die zum Ausschmelzen und/oder Ausbrennen des Modells erforderliche Temperatur vorgeheizt ist. Typischerweise erfolgt das Ausschmelzen und/oder Ausbrennen bei einer Temperatur von ca. 850°C. Vorzugsweise wird der vom Einbettmassenhersteller vorgeschlagene Temperaturzyklus verwendet. Oft ist das Modell bereits nach einer Stunde vollständig verbrannt bzw. ausgeflossen. Die maximale Verweildauer im Ofen kann je nach Einbettmasse und Hersteller variieren. In den meisten Fällen ist es ausreichend, die Form für 1 bis 8 Stunden bei der Maximaltemperatur im Ofen zu belassen. Der Aufheizvorgang kann mehrere Stunden (1-12 h) in Anspruch nehmen.

In die so hergestellte Negativform des ursprünglichen Modells wird in Schritt (D) eine Metalllegierung gegossen oder ein geeignetes Glaskeramikmaterial gepresst. Vorzugsweise werden für dentale Zwecke geeignete Metalllegierungen bzw. für dentale Zwecke geeignete Glaskeramikmaterialien verwendet.

Die Erfindung wird im Folgenden anhand von Figuren und Ausführungsbeispielen näher erläutert.
Figur 1 zeigt die thermische Ausdehnungskurve des erfindungsgemäßen Materials aus Beispiel 2, gemessen an Zylindern mit 6 mm Durchmesser und 6 mm Höhe. Die Messung wurde mit einem Messgerät des Typs Q400 der Firma TA Instruments mit Macro-Expansion Tool durchgeführt.
Figur 2 zeigt die thermische Ausdehnungskurve des erfindungsgemäßen Materials aus Beispiel 4.
Figur 3 zeigt die thermische Ausdehnungskurve des erfindungsgemäßen Materials aus Beispiel 5.
Figur 4 zeigt die thermische Ausdehnungskurve des erfindungsgemäßen Materials aus Beispiel 6.

Die Figuren 5 und 6 zeigen die Ausdehnungskurven handelsüblicher Werkstoffe gemäß dem Stand der Technik.

Die Figuren 7 und 8 zeigen die thermischen Ausdehnungskurven von Vergleichsmaterialien, die keine inerte Komponente (c) enthalten.

Die Figuren 9 bis 11 zeigen dentale Brückengerüste aus Glaskeramik, die unter Verwendung der erfindungsgemäßen Werkstoffe aus den Beispielen 4 (Fig. 9), 5 (Fig. 10) und 8 (Fig. 11) hergestellt wurden, direkt nach dem Ausbetten. Die Brückengerüste weisen keine Pressfahnen auf.

Die Figuren 12 bis 14 zeigen dentale Brückengerüste aus Glaskeramik, die unter Verwendung von Vergleichsmaterialen ohne Komponente (c) hergestellt wurden (Fig. 12, Material V1; Fig. 13, Material V2; Fig. 14, Material V3). Die Brückengerüste weisen deutlich erkennbare Pressfahnen auf.

Die Figuren 15 und 16 zeigen dentale Brückengerüste aus Glaskeramik, die unter Verwendung von handelsüblichen Werkstoffen hergestellt wurden. Die Brückengerüste weisen deutlich erkennbare Pressfahnen auf.

### Ausführungsbeispiele

### Beispiele 1 bis 9

### Modelliermaterialien

Die in Tabelle 1 aufgeführten Komponenten wurden in den angegebenen Mengen homogen miteinander gemischt. Die Komponenten wurden eingewogen, 1 Stunde bei ca. 50°C und danach bei Raumtemperatur für ca. 16h (über Nacht) gerührt. Bei pigment- und füllstoffhaltigen Zusammensetzungen wurden das Pigment bzw. der Füllstoff in UDMA eingerührt, die Massen dann dreimal über einen Dreiwalzenbock bei einer Spaltweite von 10 µm homogenisiert und dispergiert und danach in die restliche, bereits gelöste organische Matrix eingerührt (mindestens 1 Stunde bei Raumtemperatur). Schließlich wurden ggf. weitere Additive wie Verdickungsmittel zu der Paste gegeben und nochmal für mindestens 1 Stunde gerührt.

**Tabelle 1: Modelliermaterialien für die Stereolithographie**

| **Komponente** | **Zusammensetzung [Gew.-%]** | | | | | | | | |
|---|---|---|---|---|---|---|---|---|---|
| | **1** | **2** | **3** | **4** | **5** | **6** | **7** | **8** | **9** |
| **Monomer (a)** | | | | | | | | | |
| UDMA¹⁾ | 42 | 44 | 49 | 48,95 | 49 | 36,15 | 34 | 49 | 32 |
| TEGDMA²⁾ | 27 | 25 | 10,04 | 20 | 20 | 20,88 | 17 | 20 | 27 |
| Bis-GMA³⁾ | - | - | - | - | - | - | - | - | 10 |

| **Initiator (b)** | | | | | | | | | |
|---|---|---|---|---|---|---|---|---|---|
| Irgacure 819⁴⁾ | 0,95 | - | - | 0,95 | - | - | - | | - |
| TPO⁵⁾ | - | 0,95 | 0,95 | - | 0,95 | 0,92 | 0,95 | 0,95 | 0,95 |

| **Inerte Komponente (c)** | | | | | | | | | |
|---|---|---|---|---|---|---|---|---|---|
| PPG 2000⁶⁾ | 30 | 30 | 40 | 30 | 30 | 30 | - | 20 | 30 |
| PPG 1500⁷⁾ | - | - | - | - | - | - | 20 | - | - |

| **Komponente** | **Zusammensetzung [Gew.-%]** | | | | | | | | |
|---|---|---|---|---|---|---|---|---|---|
| PPG 4000⁸⁾ | - | - | - | - | - | - | 8 | - | - |
| PPG 400⁹⁾ | - | - | - | - | - | - | - | 10 | - |

| **Farbstoff** | | | | | | | | | |
|---|---|---|---|---|---|---|---|---|---|
| Sudan IV¹⁰⁾ | 0,05 | - | 0,01 | 0,05 | 0,05 | 0,05 | 0,05 | 0,05 | 0,05 |
| Sudan Black B¹¹⁾ | - | 0,05 | - | - | - | - | - | - | - |
| Weißpigment (TiO₂)¹²⁾ | - | - | - | 0,05 | - | - | - | - | - |

| **Weitere Komponenten** | | | | | | | | | |
|---|---|---|---|---|---|---|---|---|---|
| Wachspartikel¹³⁾ | - | - | - | - | - | 10 | - | - | - |
| Verdickungsmittel ¹⁴⁾ | - | - | - | - | - | 2 | - | - | - |
| PMMA-Partikel¹⁵⁾ | - | - | - | - | - | - | 20 | - | - |

| | | | | | | | | | |
|---|---|---|---|---|---|---|---|---|---|
| ¹⁾ Urethandimethacrylat (CAS-Nr. 72869-86-4) ²⁾ Triethylenglycoldimethacrylat (CAS-Nr. 109-16-0) ³⁾ Additionsprodukt aus Methacrylsäure und Bisphenol-A-diglycidylether ⁴⁾ Phenylbis(2,4,6-trimethylbenzoyl)phosphinoxid (CAS-Nr. 162881-26-7) ⁵⁾ Diphenyl(2,4,6-trimethylbenzoyl)phosphinoxid (CAS-Nr. 75980-60-8) ⁶⁾ Poly(propylenglykol) (CAS-Nr. 25322-69-4), MW = 2000 g/mol ⁷⁾ Poly(propylenglykol) (CAS-Nr. 25322-69-4), MW = 1500 g/mol ⁸⁾ Poly(propylenglykol) (CAS-Nr. 25322-69-4), MW = 4000 g/mol ⁹⁾ Poly(propylenglykol) (CAS-Nr. 25322-69-4), MW = 400g/mol ¹⁰⁾ CAS-Nr. 85-83-6 ¹¹⁾ CAS-Nr. 4197-25-5 ¹²⁾ Titandioxid Weisspigment, Partikelgröße D50 <500 nm ¹³⁾ MC6015, micronisiertes Carnaubawachs, d = 1-10 µm ¹⁴⁾ Lösung eines hochmolekularen, harnstoffmodifizierten, mittelpolaren Polyamids (Byk 430) ¹⁵⁾ hochvernetztes PMMA, Chemisnow MX80H3wT (Soken Chemical & Engineering Co., Ltd., Japan), D50 = 800 nm | | | | | | | | | |

### Beispiel 10

### Modelliermaterialien - Vergleichsbeispiele

Analog zu den Beispielen 1 bis 9 wurden die in Tabelle 2 aufgeführten Vergleichsmaterialien hergestellt.

**Tabelle 2: Vergleichsmaterialien für die Stereolithographie**

| **Komponente** | **Zusammensetzung [Gew.-%]** | | |
|---|---|---|---|
| | **V1*** | **V2*** | **V3*** |
| **Monomer (a)** | | | |
| UDMA¹⁾ | - | 74 | 40 |
| TEGDMA²⁾ | - | 25 | 25 |
| SR348C¹⁶⁾ | 59 | - | - |
| SR480¹⁷⁾ | 40 | - | - |

| **Initiator (b)** | | | |
|---|---|---|---|
| TPO⁵⁾ | 0,95 | 0,9 | 0,95 |
| **Inerte Komponente (c)** | - | - | - |

| **Farbstoff** | | | |
|---|---|---|---|
| Sudan IV¹⁰⁾ | 0,05 | 0,1 | 0,05 |

| **Weitere Komponenten** | | | |
|---|---|---|---|
| Wachspartikel¹³⁾ | - | - | 34 |

| | | | |
|---|---|---|---|
| * Vergleichsmaterial ¹⁻¹⁵⁾ siehe Tabelle 1 ¹⁶⁾ Bisphenol-A-dimethacrylat mit 3 Ethoxygruppen (Fa. Sartomer) ¹⁷⁾ Bisphenol-A-dimethacrylat; durchschnittlich 10 fach ethoxyliert | | | |

### Beispiel 11

### Messung der thermischen Ausdehnung

Zur Bestimmung der thermischen Ausdehnung der Werkstoffe wurden aus den in den Tabellen 1 und 2 beschriebenen Materialien mit einem Drucker stereolithographisch Zylinder mit 6 mm Durchmesser und 6 mm Höhe hergestellt. Die Zylinder wurden schichtweise gehärtet und anschließend in einem Nachbelichtungsgerät bei einer Wellenlänge von 400 nm mit einer Intensität von 10 mW/cm² für 5 Minuten nachbelichtet. Dann wurden die Zylinder in die Probenkammer eines thermomechanischen Analysators (Typs Q400 der Firma TA Instruments mit Macro-Expansion Tool) gestellt und mit einer Heizrate von 5 K/min auf 800°C erhitzt. Es wurde die lineare thermische Ausdehnung der Zylinder während des Aufheizens und der thermischen Zersetzung im Temperaturbereich von 30°C bis 800°C in einer Luftatmosphäre gemessen. Die Anpresskraft der Messsonde betrug 0,1 N.

In den Figuren 1 bis 4 sind die Ausdehnungskurven für die Werkstoffe aus den Beispielen 2, 4, 5 und 6 gezeigt. Die thermische Ausdehnung blieb in allen Fällen über den ganzen Messbereich deutlich unter 0,7%. Die maximale lineare thermische Ausdehnung wurde in allen Fällen bei einer Temperatur von unter 90°C erreicht. Danach blieb die Länge der Zylinder unterhalb des Maximalwerts. Ab 300°C zersetzten sich die Materialein thermisch und brannten rückstandslos aus (Glührückstand in allen Fällen < 0,1 Gew.-%). Die Zersetzung äußert sich in einem starken Abfall der linearen thermischen Ausdehnung.

Das Beispiel 6 zeigt, dass die als Füllstoff eingesetzten Wachspartikel praktisch keinen Einfluss auf die Ausdehnung haben.

Die Ausdehnungskurven der übrigen Beispiele verlaufen ähnlich. In Beispiel 8 wird die maximale thermische Ausdehnung von < 1,5 % bei 125°C erreicht.

Zum Vergleich wurden die Ausdehnungskurven herkömmlicher, kommerziell erhältlicher Stereolithographie-Werkstoffe für die Lost-Wax Technik ermittelt. Figur 5 zeigt die Ausdehnungskurve des Materials Bego VarseoWax CAD-Cast. Das Material dehnt sich um mehr als 4% aus, und die Temperatur der maximalen Ausdehnung wird erst kurz vor der thermischen Zersetzung, d.h. bei mehr als 300°C erreicht.

In Figur 6 ist die Ausdehnungskurve eines weiteren handelsüblichen Stereolithographie-Harzes für die Lost-Wax Technik (3D Systems Visijet FTX-Cast) gezeigt. Das Material dehnt sich um mehr als 2% aus, und die Temperatur der maximalen thermischen Ausdehnung liegt über 200°C.

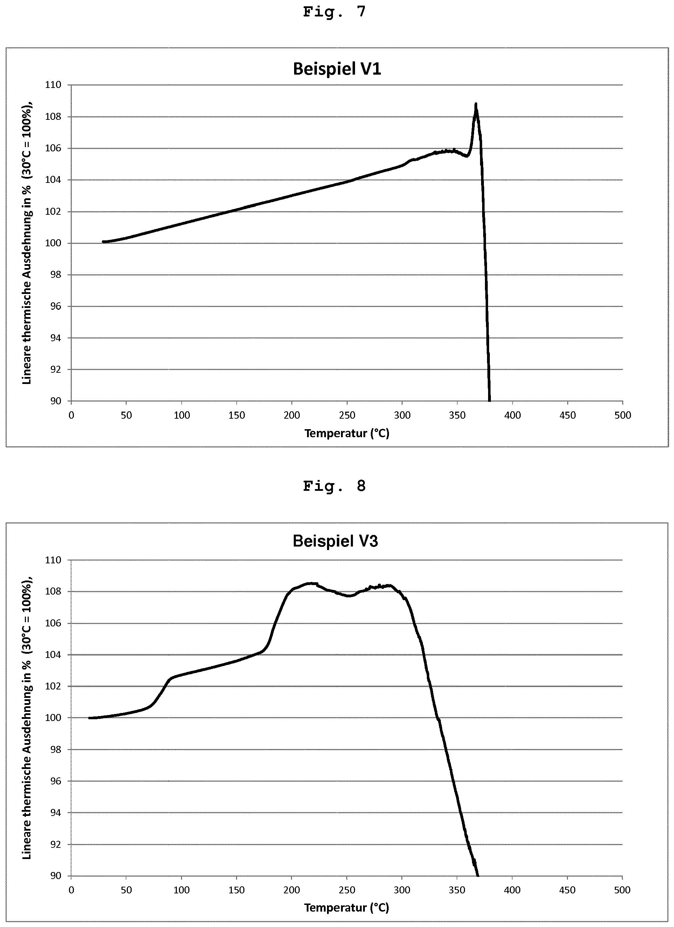

Die Figuren 7 und 8 zeigen die Ausdehnungskurven der Vergleichsmaterialien V1 und V3. Das Material V1 dehnte sich um mehr als 6% aus (Fig. 7), das Material V3 um mehr als 8% (Fig. 8). Die Temperatur der maximalen Ausdehnung wurde in beiden Fällen erst kurz vor der thermischen Zersetzung erreicht, d.h. bei über 300°C bzw. über 200°C.

Die Messungen zeigen, dass die maximale thermische Ausdehnung der Vergleichsmaterialien in allen Fällen größer als die der erfindungsgemäßen Materialien ist. Durch die Zugabe der Komponente (c) kann die thermische Ausdehnung wirksam verringert werden. Außerdem liegt bei den Vergleichsmaterialen das Maximum der linearen thermischen Ausdehnung bei deutlich höheren Temperaturen, die durch die Zersetzungstemperatur der Materialien bestimmt werden.

### Beispiel 12

### Herstellung von Modellen

Mit einem 3D-Drucker wurden aus den in den Beispielen 1 bis 9 beschriebenen Materialien und mit handelsüblichen Werkstoffen Modelle einer dreigliedrigen Brücke gefertigt. Zur Herstellung der Modelle wurde in allen Fällen der gleiche Datensatz verwendet.

Die Modelle wurden auf einer Muffelbasis mit der passenden Muffellehre aufgewachst und mit Presskanälen versehen. Anschließend wurden die Modelle jeweils in eine handelsübliche Einbettmasse auf Phosphatbasis eingebettet (200 g PressVest Speed; Fa. Ivoclar Vivadent AG). Die Feineinbettung der Kavitäten wurde mit einem kleinen Pinsel vorgenommen. Die Eigebettete Muffel wurde vibrationsfrei für 35 Minuten abbinden gelassen. Danach wurden die Muffeln direkt in den auf 850°C vorgewärmten Vorwärmofen gestellt und dort für 1,5 h bei 850°C belassen, um die Modelle komplett daraus zu entfernen. Anschließend wurden die Muffeln aus dem Vorwärmofen entnommen, die heißen Muffeln mit einem Keramikrohling (IPS e.max Press Rohling, Fa. Ivoclar Vivadent AG) bestückt, in den heißen Pressofen (Programat EP 5010, Fa. Ivoclar Vivadent) gestellt und das gewählte Pressprogramm gestartet.

Nach dem Ende des Pressvorgangs wurden die Muffeln aus dem Ofen genommen und zum Abkühlen an einem vor Zugluft geschützten Platz auf ein Abkühlgitter gestellt. Nach Abkühlen auf Raumtemperatur wurden die Muffeln mit einer Trennscheibe separiert und die Pressobjekte ausgebettet. Die Grobausbettung erfolgte mit Glanzstrahlmittel bei 4 bar Druck, die die Feinausbettung mit Glanzstrahlmittel bei 2 bar Druck. Die Pressergebnisse wurden direkt nach der Feinausbettung bewertet.

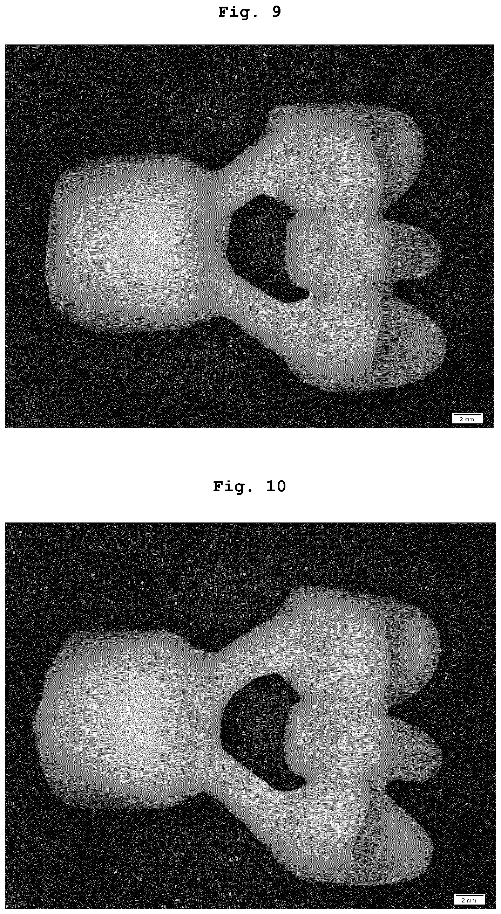

Die Figuren 9 bis 11 zeigen beispielhaft Brückengerüste, die unter Verwendung der erfindungsgemäßen Werkstoffe aus den Beispielen 4, 5 und 8 hergestellt wurden. Die Aufnahmen wurden direkt nach dem Ausbetten angefertigt. Es sind keine Pressfahnen sichtbar. Die mit den anderen erfindungsgemäßen Werkstoffen hergestellten Brücken waren ähnlich, in keinem Fall waren Pressfahnen vorhanden.

Im Gegensatz dazu wiesen die unter Verwendung der Vergleichsmaterialein gefertigten Brücken deutlich erkennbare Pressfahnen auf, die auf Risse in der Gießform zurückzuführen sind, die bei der Ausdehnung der Modelliermaterialen entstanden.

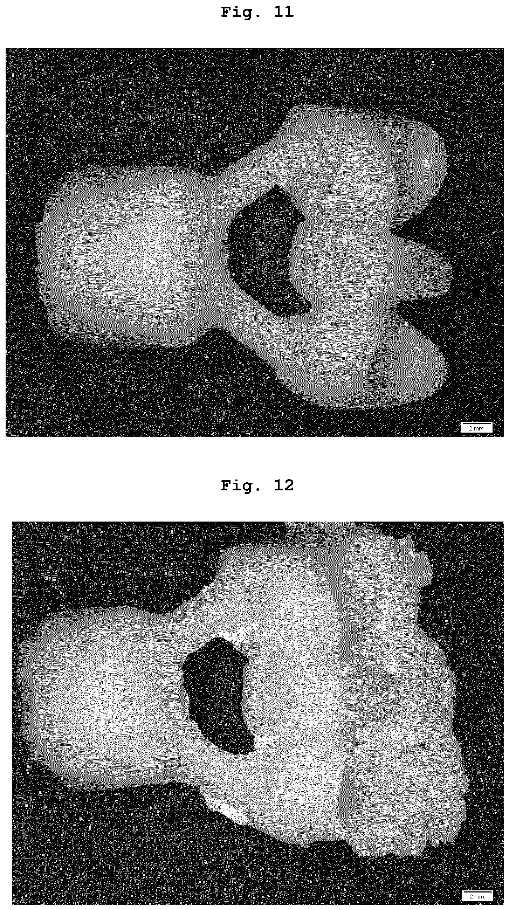

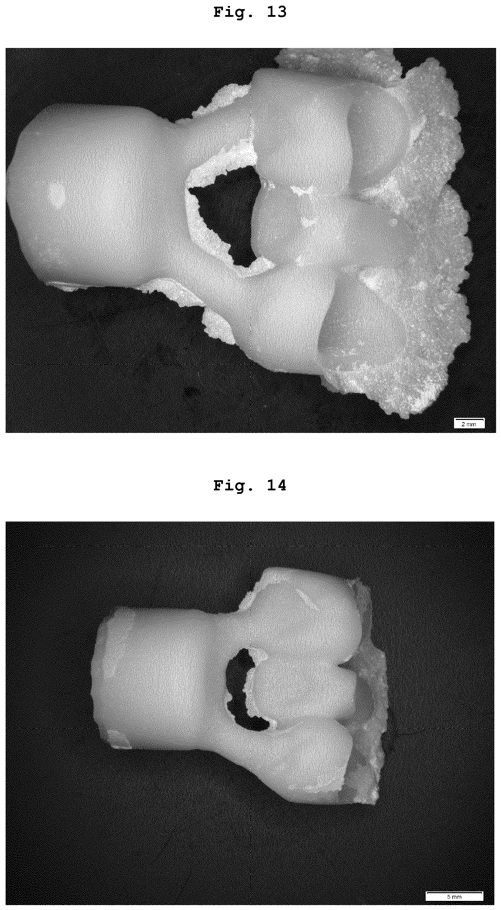

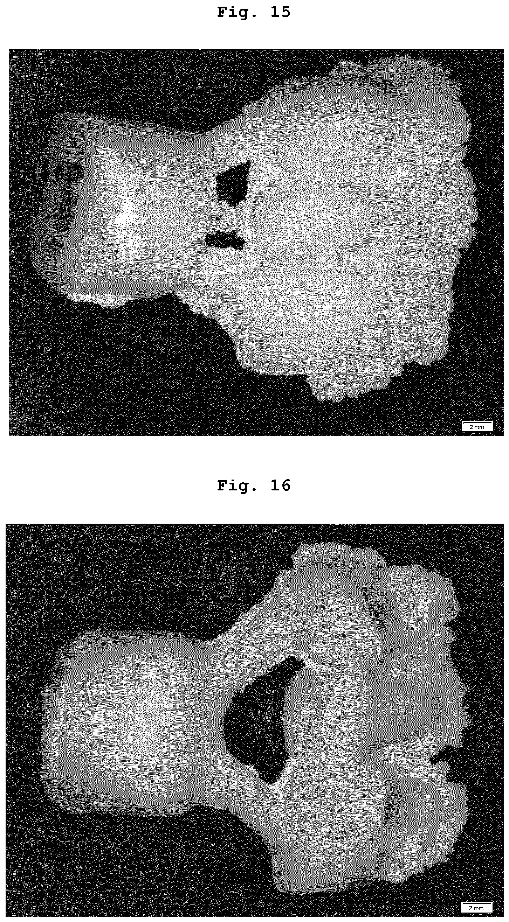

Die Figuren 12 bis 14 zeigen die unter Verwendung der Vergleichsmaterialen V1 bis V3 erhaltenen Brückengerüste. Figur 15 zeigt eine unter Verwendung des handelsübliche Materials Bego Varseo Wax CAD-Cast und Figur 16 eine unter Verwendung des Produkts 3D Systems Visijet FTX-Cast gefertigte Brücke. In allen Fällen sind Pressfahnen vorhanden, die eine mehr oder weniger aufwendige Nachbearbeitung der Restaurationen erforderlich machen. Zum Teil waren die Restaurationen unbrauchbar.

Selbst die unter Verwendung des Produkts 3D Systems Visijet FTX-Cast erhaltende Brücke wies Pressfahnen auf, obwohl dieses Material mit ca. 2% eine relativ geringe maximale thermische Ausdehnung hat. Im Gegensatz dazu war die mit dem erfindungsgemäßen Material aus Beispiel 8 erhaltene Brücke (Fig. 11; max. Ausdehnung < 1,5%) fehlerfrei. Die maximale thermische Ausdehnung wird im Fall des erfindungsgemäßen Materials bei 125°C erreicht, im Fall des Vergleichsmaterials erst bei über 200°C.

Das Vergleichsmaterial V3 enthielt Wachspartikel aber keine Komponente (c). Das Modell dehnte sich beim Ausbrennen stark aus, die Pressergebnisse waren dementsprechend schlecht.

## Patentansprüche

1. Radikalisch polymerisierbare Zusammensetzung, die
(a) mindestens ein radikalisch polymerisierbares Monomer,
(b) mindestens einen Initiator für die radikalische Polymerisation und
(c) mindestens eine inerte Komponente enthält,
**dadurch gekennzeichnet, dass** die Zusammensetzung 20 bis 90 Gew.-% radikalisch polymerisierbares Monomer(a), 0,1 bis 5 Gew.-% Initiator (b) und 9,9 bis 79,9 Gew.-% inerte Komponente (c) enthält, jeweils bezogen auf die Gesamtmasse der Zusammensetzung, dass die Zusammensetzung als Komponente (a) mindestens ein (Meth)acrylat und/oder (Meth)acrylamid enthält und dass die inerte Komponente (c) aus
- Polyethylenglykolen (PEG) mit einem Molekulargewicht von 1000 bis 5000 g/mol,
- Polypropylenglykolen (PPG) mit einem Molekulargewicht von 1000 bis 4000 g/mol,
- PEG-PPG-Co-Polymeren mit einem Molekulargewicht von 1000 bis 10000 g/mol,
- Ethylendiamintetrakis-propoxylaten und Ethylendiamintetrakis-ethoxylaten mit einem Molekulargewicht von 1000 bis 10000 g/mol ausgewählt ist.

2. Zusammensetzung nach Anspruch 1, in der die inerte Komponente (c) aus
- Polyethylenglykolen (PEG) mit einem Molekulargewicht von 1000 bis 3000 g/mol,
- Polypropylenglykolen (PPG) mit einem Molekulargewicht von 1500 bis 3000 g/mol,
- PEG-PPG-Co-Polymeren mit einem Molekulargewicht von 1500 bis 5000 g/mol,
- Ethylendiamintetrakis-propoxylaten und Ethylendiamintetrakis-ethoxylaten mit einem Molekulargewicht von 1000 bis 5000 g/mol ausgewählt ist.

3. Zusammensetzung nach Anspruch 1 oder 2, die als Komponente (a) ein oder mehrere mono- oder multifunktionelle (Meth)acrylate oder eine Mischung davon enthält.

4. Zusammensetzung nach Anspruch 3, die als Komponente (a) UDMA, TEGDMA, Bis-GMA (Additionsprodukt aus Methacrylsäure und Bisphenol-A-diglycidylether) oder 2-Phenoxyethylacrylat, eine Mischung von UDMA, TEGDMA und 2-Phenoxythylacrylat oder eine Mischung von UDMA, TEGDMA und Bis-GMA oder eine Mischung von UDMA und TEGDMA enthält.

5. Zusammensetzung nach Anspruch 4, die als Komponente (a) UDMA, TEGDMA oder eine Mischung davon und maximal 20 Gew.-% weitere Monomere enthält, bezogen auf die Gesamtmasse der Komponente (a).

6. Zusammensetzung nach einem der Ansprüche 1 bis 5, die als Initiator (b) einen Photoinitiator enthält.

7. Zusammensetzung nach Anspruch 6, die als Photoinitiator Benzophenon, Benzoin, ein α-Diketon, 9,10-Phenanthrenchinon, 1-Phenyl-propan-1,2-dion, Diacetyl, 4,4'-Dichlorbenzil, Campherchinon (CQ), 2,2-Dimethoxy-2-phenylacetophenon, ein α-Diketon in Kombination mit einem Amin als Reduktionsmittel, einen Norrish-Typ-I-Photoinitiator, ein Monoacyl- oder Bisacylphosphinoxid, eine Monoacyltrialkyl- oder Diacyldialkylgermanium-Verbindung, Benzoyltrimethylgermanium, Dibenzoyldiethylgermanium, Bis(4-methoxybenzoyl)diethylgermanium (MBDEGe), eine Mischung von Bis(4-methoxybenzoyl)diethylgermanium in Kombination mit Campherchinon und 4-Dimethylaminobenzoesäureethylester, Campherchinon (CAS Nr. 10373-78-1) in Kombination mit Ethyl-4-(dimethylamino)benzoat (EMBO, CAS Nr. 10287-53-3), 2,4,6-Trimethylbenzoyldipenylphosphinoxid (CAS Nr. 75980-60-8), Ethyl(2,4,6-trimethylbenzoyl)phenylphosphinat (CAS Nr. 84434-11-7), Phenylbis(2,4,6-trimethylbenzoyl)-phosphinoxid (CAS 162881-26-7), Bis(2,6-difluoro-3-(1-hydropyrrol-1-yl)phenyl)titanocen (CAS Nr. 125051-32-3), 2-Benzyl-2-(dimethylamino)-4'-morpholinobutyrophenon (CAS Nr. 119313-12-1) und/oder 1-Butanon-2-(dimethylamino)-2-(4-methylphenyl)methyl-1-4-(4-morpholinyl)phenyl (CAS-Nr. 119344-86-4) enthält.

8. Zusammensetzung nach einem der Ansprüche 1 bis 7, die zusätzlich mindestens ein Farbmittel und/oder einen Inhibitor enthält.

9. Zusammensetzung nach einem der Ansprüche 1 bis 8, die
- 42,5 bis 82,5 Gew.-% Komponente (a),
- 0,3 bis 2,5 Gew.-% (Photo-)-Initiator (b) und
- 15 bis 55 Gew.-% inerte Komponente (c) enthält,
jeweils bezogen auf die Gesamtmasse der Zusammensetzung.

10. Zusammensetzung nach Anspruch 9, die
- 54,7 bis 77,7 Gew.-% Komponente (a),
- 0,5 bis 1,5 Gew.-% (Photo-)Initiator (b) und
- 23,8 bis 44,8 Gew.-% inerte Komponente (c) enthält,
jeweils bezogen auf die Gesamtmasse der Zusammensetzung.

11. Zusammensetzung nach Anspruch 10, die
- 64 bis 74 Gew.-% UDMA, TEGDMA, eine Mischung von UDMA und TEGDMA oder eine Mischung von UDMA, TEGDMA und Bis-GMA als Komponente (a),
- 0,6 bis 1,2 Gew.-% Photoinitiator (b) und
- 24,8 bis 35,4 Gew.-% Polypropylenglycol (PPG) mit einem Molekulargewicht von 1000 bis 4000 g/mol als inerte Komponente (c) enthält,
jeweils bezogen auf die Gesamtmasse der Zusammensetzung.

12. Zusammensetzung nach einem der Ansprüche 9 bis 11, die zusätzlich
- 0,0001 bis 1 Gew.-% Farbmittel; und/oder
- 0,0001 bis 2 Gew.-% UV-Absorber; und/oder
- 0 bis 40 Gew.-% organischen Füller; und/oder
- 0 bis 5 Gew.-% weitere(s) Additiv(e) enthält,
jeweils bezogen auf die Gesamtmasse der Zusammensetzung.

13. Zusammensetzung nach einem der Ansprüche 1 bis 12, die eine maximale lineare thermische Ausdehnung unter 1,5% aufweist.

14. Zusammensetzung nach Anspruch 13, bei der die maximale lineare thermische Ausdehnung bei einer Temperatur von unter 150°C erreicht wird.

15. Verwendung einer Zusammensetzung gemäß einem der Ansprüche 1 bis 14 zur Herstellung eines Modells einer Dentalrestauration durch Stereolithographie.

16. Verwendung nach Anspruch 15, bei dem die Dentalrestauration ein Inlay, Onlay, Veneer, eine Krone, Brücke, ein Gerüst oder eine abnehmbare (Teil-)Prothese ist.

## Claims

1. Radically polymerizable composition which comprises
(a) at least one radically polymerizable monomer,
(b) at least one initiator for the radical polymerization and
(c) at least one inert component,
**characterized in that** the composition comprises 20 to 90 wt.-% radically polymerizable monomer (a), 0.1 to 5 wt.-% initiator (b) and 9.9 to 79.9 wt.-% inert component (c), in each case relative to the total mass of the composition, that the composition comprises, as component (a), at least one (meth)acrylate and/or (meth)acrylamide, and that the inert component (c) is selected from
- polyethylene glycols (PEG) with a molecular weight of 1000 to 5000 g/mol
- polypropylene glycols (PPG) with a molecular weight of 1000 to 4000 g/mol
- PEG-PPG copolymers with a molecular weight of 1000 to 10,000 g/mol
- ethylenediamine tetrakispropoxylates and ethylenediamine tetrakisethoxylates with a molecular weight of 1000 to 10,000 g/mol.

2. Composition according to claim 1, in which the inert component (c) is selected from
- polyethylene glycols (PEG) with a molecular weight of 1000 to 3000 g/mol,
- polypropylene glycols (PPG) with a molecular weight of 1500 to 3000 g/mol,
- PEG-PPG copolymers with a molecular weight of 1500 to 5000 g/mol,
- ethylenediamine tetrakispropoxylates and ethylenediamine tetrakisethoxylates with a molecular weight of 1000 to 5000 g/mol.

3. Composition according to claim 1 or 2, which comprises, as component (a), one or more mono- or multifunctional (meth)acrylates or a mixture thereof.

4. Composition according to claim 3, which comprises, as component (a), UDMA, TEGDMA, bis-GMA (addition product of methacrylic acid and bisphenol A diglycidyl ether) or 2-phenoxyethyl acrylate, a mixture of UDMA, TEGDMA and 2-phenoxyethyl acrylate or a mixture of UDMA, TEGDMA and bis-GMA or a mixture of UDMA and TEGDMA.

5. Composition according to claim 4, which comprises, as component (a), UDMA, TEGDMA or a mixture thereof and at most 20 wt.-% of further monomers, relative to the total mass of component (a).

6. Composition according to one of claims 1 to 5, which comprises, as initiator (b), a photoinitiator.

7. Composition according to claim 6, which comprises, as photoinitiator, benzophenone, benzoin, an α-diketone, 9,10-phenanthrenequinone, 1-phenyl-propane-1,2-dione, diacetyl, 4,4'-dichlorobenzil, camphorquinone (CQ), 2,2-dimethoxy-2-phenyl-acetophenone, an α-diketone in combination with an amine as reducing agent, a Norrish type I photoinitiator, a monoacyl- or bisacylphosphine oxide, a monoacyltrialkyl- or diacyldialkylgermanium compound, benzoyltrimethylgermanium, dibenzoyldiethylgermanium, bis(4-methoxybenzoyl)diethylgermanium (MBDEGe), a mixture of bis(4-methoxybenzoyl)diethylgermanium in combination with camphorquinone and 4-dimethylaminobenzoic acid ethyl ester, camphorquinone (CAS No. 10373-78-1) in combination with ethyl 4-(dimethylamino)benzoate (EMBO, CAS No. 10287-53-3), 2,4,6-trimethylbenzoyl diphenylphosphine oxide (CAS No. 75980-60-8), ethyl (2,4,6-trimethylbenzoyl) phenylphosphinate (CAS No. 84434-11-7), phenylbis(2,4,6-trimethylbenzoyl)phosphine oxide (CAS 162881-26-7), bis(2,6-difluoro-3-(1-hydropyrrol-1-yl)phenyl)titanocene (CAS No. 125051-32-3), 2-benzyl-2-(dimethylamino)-4'-morpholinobutyrophenone (CAS No. 119313-12-1), and/or 1-butanone, 2-(dimethylamino)-2-(4-methylphenyl)methyl-1-4-(4-morpholinyl)phenyl (CAS No. 119344-86-4).

8. Composition according to one of claims 1 to 7, which additionally comprises at least one colorant and/or one inhibitor.

9. Composition according to one of claims 1 to 8, which comprises
- 42.5 to 82.5 wt.-% component (a),
- 0.3 to 2.5 wt.-% (photo)initiator (b) and
- 15 to 55 wt.-% inert component (c),
in each case relative to the total mass of the composition.

10. Composition according to claim 9, which comprises
- 54.7 to 77.7 wt.-% component (a),
- 0.5 to 1.5 wt.-% (photo)initiator (b) and
- 23.8 to 44.8 wt.-% inert component (c),
in each case relative to the total mass of the composition.

11. Composition according to claim 10, which comprises
- 64 to 74 wt.-% UDMA, TEGDMA, a mixture of UDMA and TEGDMA or a mixture of UDMA, TEGDMA and bis-GMA as component (a),
- 0.6 to 1.2 wt.-% photoinitiator (b) and
- 24.8 to 35.4 wt.-% polypropylene glycol (PPG) with a molecular weight of 1000 to 4000 g/mol as inert component (c),
in each case relative to the total mass of the composition.

12. Composition according to one of claims 9 to 11, which additionally comprises
- 0.0001 to 1 wt.-% colorant; and/or
- 0.0001 to 2 wt.-% UV absorber; and/or
- 0 to 40 wt.-% organic filler; and/or
- 0 to 5 wt.-% further additive(s),
in each case relative to the total mass of the composition.

13. Composition according to one of claims 1 to 12, which has a maximum linear thermal expansion below 1.5%.

14. Composition according to claim 13, in which the maximum linear thermal expansion is reached at a temperature of below 150°C.

15. Use of a composition according to one of claims 1 to 14 for the production of a model of a dental restoration by stereolithography.

16. Use according to claim 15, in which the dental restoration is an inlay, onlay, veneer, a crown, bridge, a framework or a removable (partial) prosthesis.

## Revendications

1. Composition polymérisable par voie radicalaire, qui contient
(a) au moins un monomère polymérisable par voie radicalaire,
(b) au moins un amorceur pour la polymérisation radicalaire et
(c) au moins un composant inerte,
**caractérisé en ce que** la composition contient 20 à 90 % en poids de monomère (a) polymérisable par voie radicalaire, 0,1 à 5 % en poids d'amorceur (b) et 9,9 à 79,9 % en poids de composant inerte (c), chaque fois par rapport à la masse totale de la composition, **en ce que** la composition contient en tant que composant (a) au moins un (méth)acrylate et/ou (méth)acrylamide et **en ce que** le composant inerte (c) est choisi parmi
- les polyéthylèneglycols (PEG) ayant une masse moléculaire de 1 000 à 5 000 g/mole,
- les polypropylèneglycols (PPG) ayant une masse moléculaire de 1 000 à 4 000 g/mole,
- les copolymères PEG-PPG ayant une masse moléculaire de 1 000 à 10 000 g/mole,
- les éthylènediaminetétrakis-propoxylates et éthylènediaminetétrakis-éthoxylates ayant une masse moléculaire de 1 000 à 10 000 g/mole.

2. Composition selon la revendication 1, dans laquelle le composant inerte (c) est choisi parmi
- les polyéthylèneglycols (PEG) ayant une masse moléculaire de 1 000 à 3 000 g/mole,
- les polypropylèneglycols (PPG) ayant une masse moléculaire de 1 500 à 3 000 g/mole,
- les copolymères PEG-PPG ayant une masse moléculaire de 1 500 à 5 000 g/mole,
- les éthylènediaminetétrakis-propoxylates et éthylènediaminetétrakis-éthoxylates ayant une masse moléculaire de 1 000 à 5 000 g/mole.

3. Composition selon la revendication 1 ou 2, qui contient en tant que composant (a) un ou plusieurs (méth)acrylate(s) mono- ou multifonctionnel(s) ou un mélange de tels (méth)acrylates.

4. Composition selon la revendication 3, qui contient en tant que composant (a) de l'UDMA, du TEGDMA, du bis-GMA (produit d'addition d'acide méthacrylique et d'éther diglycidylique de bisphénol A) ou de l'acrylate de 2-phénoxyéthyle, un mélange d'UDMA, de TEGDMA et d'acrylate de 2-phénoxyéthyle ou un mélange d'UDMA, de TEGDMA et de bis-GMA ou un mélange d'UDMA et de TEGDMA.

5. Composition selon la revendication 4, qui contient en tant que composant (a) de l'UDMA, du TEGDMA ou un mélange de ceux-ci et au maximum 20 % en poids d'autres monomères, par rapport à la masse totale du composant (a).

6. Composition selon l'une quelconque des revendications 1 à 5, qui contient en tant qu'amorceur (b) un photoamorceur.

7. Composition selon la revendication 6, qui contient en tant que photoamorceur de la benzophénone, de la benzoïne, une α-dicétone, de la 9,10-phénanthrènequinone, de la 1-phényl-propane-1,2-dione, du diacétyle, du 4,4'-dichlorobenzile, de la camphoquinone (CQ), de la 2,2-diméthoxy-2-phényl-acétophénone, une α-dicétone en association avec une amine en tant que réducteur, un photoamorceur de type Norrish I, un oxyde de monoacyl- ou bisacylphosphine, un composé monoacyltrialkyl- ou diacyldialkylgermanium, du benzoyltriméthyl-germanium, du dibenzoyldiéthylgermanium, du bis(4-méthoxybenzoyl)diéthyl-germanium (MBDEGe), un mélange de bis(4-méthoxybenzoyl)diéthylgermanium en association avec de la camphoquinone et du 4-diméthylaminobenzoate d'éthyle, de la camphoquinone (n° CAS 10373-78-1) en association avec du 4-(diméthylamino)-benzoate d'éthyle (EMBO, n° CAS 10287-53-3), de l'oxyde de 2,4,6-triméthyl-benzoyldiphénylphosphine (n° CAS 7598060-8), du (2,4,6-triméthylbenzoyl)phényl-phosphinate d'éthyle (n° CAS 84434-11-7), de l'oxyde de phénylbis(2,4,6-triméthylbenzoyl)-phosphine (CAS 162881-26-7), du bis(2,6-difluoro-3-(1-hydropyrrol-1-yl)phényl)titanocène (n° CAS 125051-32-3), de la 2-benzyl-2-(diméthylamino)-4'-morpholinobutyrophénone (n° CAS 119313-12-1) et/ou du 1-butanone-2-(diméthylamino)-2-(4-méthylphényl)méthyl-1-4-(4-morpholinyl)phényle (n° CAS 119344-86-4).

8. Composition selon l'une quelconque des revendications 1 à 7, qui contient en outre au moins un agent colorant et/ou un inhibiteur.

9. Composition selon l'une quelconque des revendications 1 à 8, qui contient
- 42,5 à 82,5 % en poids de composant (a),
- 0,3 à 2,5 % en poids de (photo)amorceur (b) et
- 15 à 55 % en poids de composant inerte (c),
chaque fois par rapport à la masse totale de la composition.

10. Composition selon la revendication 9, qui contient
- 54,7 à 77,7 % en poids de composant (a),
- 0,5 à 1,5 % en poids de (photo)amorceur (b) et
- 23,8 à 44,8 % en poids de composant inerte (c),
chaque fois par rapport à la masse totale de la composition.

11. Composition selon la revendication 10, qui contient
- 64 à 74 % en poids d'UDMA, de TEGDMA, d'un mélange d'UDMA et de TEGDMA ou d'un mélange d'UDMA, de TEGDMA et de bis-GMA en tant que composant (a),
- 0,6 à 1,2 % en poids de photoamorceur (b) et
- 24,8 à 35,4 % en poids de polypropylèneglycol (PPG) ayant une masse moléculaire de 1 000 à 4 000 g/mole en tant que composant inerte (c),
chaque fois par rapport à la masse totale de la composition.

12. Composition selon l'une quelconque des revendications 9 à 11, qui contient en outre
- 0,0001 à 1 % en poids d'agent colorant ; et/ou
- 0,0001 à 2 % en poids d'absorbeur UV ; et/ou
- 0 à 40 % en poids de charges organiques ; et/ou
- 0 à 5 % en poids d'autre(s) additif(s),
chaque fois par rapport à la masse totale de la composition.

13. Composition selon l'une quelconque des revendications 1 à 12, qui présente une dilatation thermique linéaire maximale inférieure à 1,5 %.

14. Composition selon la revendication 13, dans laquelle la dilatation thermique linéaire maximale est atteinte à une température inférieure à 150 °C.

15. Utilisation d'une composition selon l'une quelconque des revendications 1 à 14 pour la fabrication d'un modèle d'une restauration dentaire par stéréolithographie.

16. Utilisation selon la revendication 15, dans laquelle la restauration dentaire est un inlay, un onlay, un placage, une couronne, un bridge, une armature ou une prothèse (partielle) amovible.
